# EUROPÄISCHE PATENTSCHRIFT

(11) **EP 1 276 520 B1**
(45) Veröffentlichungstag und Bekanntmachung des Hinweises auf die Patenterteilung: **20.02.2008**
(21) Anmeldenummer: 01921352.9
(22) Anmeldetag: 27.03.2001
(51) Int. Cl.: A61M 1/10

(54) **PARAKARDIALE BLUTPUMPE**
PARACARDIAL BLOOD PUMP
POMPE A SANG PARACARDIAQUE

(30) Priorität: 01.04.2000 DE 10016422
(43) Veröffentlichungstag der Anmeldung: 22.01.2003
(73) Patentinhaber: Abiomed Europe GmbH, 52074 Aachen (DE)
(72) Erfinder: SIESS, Thorsten, 52146 Würselen (DE)
(74) Vertreter: Selting, Günther
(86) Internationale Anmeldenummer: PCT/EP2001/003467
(87) Internationale Veröffentlichungsnummer: WO 2001/074419

(56) Entgegenhaltungen:
- EP-A- 0 764 448
- WO-A-00/37139
- WO-A-97/49439
- US-A- 5 824 070

## Beschreibung

Die Erfindung betrifft eine parakardiale Blutpumpe mit einem rohrförmigen Gehäuse, in dem in einem längslaufenden Strömungskanal eine Antriebseinheit für ein Pumpenrad untergebracht ist, wobei der Strömungskanal mit einem seitlich abgehenden Auslaßrohr in Verbindung steht.

Eine parakardiale Blutpumpe ist eine Blutpumpe, die Blut aus einem Teil des Herzens ansaugt und in die Aorta oder ein anderes Zielgebiet fördert, wobei das Gehäuse der Blutpumpe außen an das Herz angelegt wird, während der Pumpeneinlaß eine direkte Verbindung zu derjenigen Herzkammer hat, aus der angesaugt wird. Aus WO 97/49439 ist eine thorakale Blutpumpe bekannt. Diese Blutpumpe weist ein rohrförmiges Gehäuse auf, in welchem sich eine elektromotorische Antriebseinheit befindet. Die Antriebseinheit ist in einem Strömungskanal angeordnet, in welchem sie von Blut umströmt wird. Das Blut wird von einem eingangsseitig angeordneten Pumpenrad in einer Schraubenbewegung angetrieben und zirkuliert in dem Strömungskanal, um durch ein seitlich abgehendes Auslaßrohr das Gehäuse zu verlassen. Die Blutpumpe mit dem Gehäuse und dem Auslaßrohr ist insgesamt L-förmig ausgebildet, wodurch es möglich ist, sie an einen an der Herzwand geschaffenen Zugang anzusetzen, während das Auslaßrohr mit dem Zielgefäß verbunden wird. Derartige Blutpumpen dienen der Herzunterstützung. Sie werden temporär an das Herz angesetzt, jedoch nicht insgesamt in das Herz eingeführt, wie intrakardiale Blutpumpen, die aus WO 98/43688 bekannt sind. Bei einer intrakardialen Blutpumpe ist kein zusätzliches Gehäuse vorgesehen, in welchem die Antriebseinheit untergebracht ist. Vielmehr fördert das Pumpenrad den Blutstrom, ohne dass dieser zusammengefaßt und gezielt abgeleitet würde.

In EP 0 764 448 A2 ist eine intracardiale Pumpe beschrieben, die einen axialen Einlass und einen radialen Einlass aufweist, während der Pumpenauslass axial gerichtet ist. Eine derartige Pumpe eignet sich zur Herzunterstützung, wobei die gesamte Pumpe mit den beiden Einlässen und dem Auslass im Inneren des Herzens angeordnet ist. Keiner der beiden Einlässe weist eine Kanüle auf. Eine flexible Kanüle ist dagegen an dem Auslassende der Pumpe angeordnet, um das gepumpte Blut weit in die Arterie hineinzutragen. Der Außendurchmesser der rohrförmigen Pumpe liegt im Bereich von 9-12 mm und der Durchmesser der Kanüle ist etwa gleich groß wie derjenige des Gehäuses. Es handelt sich nicht um eine parakardiale Blutpumpe, bei der das Gehäuse der Blutpumpe außen an das Herz angelegt wird und das rohrförmige Gehäuse durch eine Punktionsstelle der Herzwand hindurch geführt werden kann.

Der Erfindung liegt die Aufgabe zugrunde, eine parakardiale Blutpumpe zu schaffen, die zur Herzunterstützung von außen an das Herz angesetzt werden kann, ohne dass die Herzwand durch Entnahme von Wandmaterial beschädigt werden müßte.

Die Lösung dieser Aufgabe erfolgt erfindungsgemäß mit den im Anspruch 1 angegebenen Merkmalen. Hiernach ist vor dem Einlass des Pumpenringes eine Kanüle angeordnet, deren Außendurchmesser annähernd so groß ist wie derjenige des die Antriebseinheit und den Strömungskanal enthaltenden Gehäuses. Die Außendurchmesser von Kanüle und Gehäuse betragen maximal 13 mm, vorzugsweise maximal 12 mm und insbesondere maximal 11 mm. Die Außendurchmesser von Gehäuse und Kanüle sind annähernd gleich, was bedeutet, dass ihre Abweichung maximal 15 %, insbesondere maximal 10 %, beträgt. Bei Förderraten von etwa 4 bis 6 Litern pro Minute ergibt ein Innendurchmesser von 6 bis 8 mm im Ansaugbereich eine Strömungsgeschwindigkeit von weniger als 1,0 m/s. Diese Strömungsgeschwindigkeit erhöht sich im Bereich des Pumpenrads auf 1,5 bis 3,0 m/s und im Bereich des ringförmigen Strömungskanals verringert sie sich dann wieder auf weniger als 1,0 m/s. Derartige Strömungsgeschwindigkeiten sind einerseits bei günstiger Strömungsführung nicht so hoch, dass eine Blutschädigung auftreten würde und andererseits sind sie groß genug, um Totwassergebiete und Thrombosen bedingt durch das starke Auswaschen sicher zu vermeiden.

Da die Blutpumpe herznah plaziert wird, kann sie eine Kanüle relativ geringer Länge haben. Durch eine kurze Kanüle werden die Strömungsverluste im Kanülenbereich vermindert, wobei die Pumpe trotz geringer Abmaße eine hohe hydraulische Leistung haben kann.

Die Pumpe hat hohe Strömungsgeschwindigkeiten im Bereich des Strömungskanals und im Kanülenbereich. Diese hohen Strömungsgeschwindigkeiten führen zu gutem Auswaschen aller blutführenden Bereiche, mit der Folge einer reduzierten Gefahr der Thrombenbildung. Dies ist dann wichtig, wenn die Pumpe über längere Zeit (bis zu einem Monat) eingesetzt wird, weil Antikoagulanz-Medikamente in geringerem Maße eingesetzt werden müssen. Hierdurch kann die Neigung zur postoperativen Blutung und zu hohem Blutverlust deutlich reduziert werden.

Die geringen Strömungsquerschnitte speziell im Bereich des Pumpenrades haben Fluiddioden-Charakter. Dies bedeutet, dass selbst bei Pumpenstillstand der Rückfluß durch die Pumpe gering ist, vorzugsweise kleiner als 1,0 1/min. Dieser geringe Rückfluß sollte sogar .bei Kanülierung vom Vorhof zur Aorta erreichbar sein, bei welcher relativ hohe physiologsche Drücke von 80 bis 100 mm Hg auftreten Bekannte vergleichbare Pumpen haben dagegen retrograde Flüsse bis zu 3 1/min bei physiologischen Druckverhältnissen.

Die erfindungsgemäße Blutpumpe ist so ausgelegt, dass sie das nötige Fördervolumen liefert und andererseits derart durch die Herzwand hindurchgeführt werden kann, dass das in der Herzwand zu erzeugende Loch ohne Materialentnahme erzeugt werden kann. Kanüle und Gehäuse können durch eine Punktionsstelle der Herzzwand hindurchgeschoben werden, die dann das Gehäuse fest umschließt, so dass die Pumpe Blut aus dem Herzen heraus fördert. Wird die Pumpe wieder herausgezogen, dann schließt sich die Herzwand an der Punktionstelle wieder bzw. sie wird an dieser Stelle geklammert oder genäht. Die erfindungsgemäße Blutpumpe ist von der Kanülenspitze an bis zum abzweigenden Auslaßrohr insgesamt zylindrisch und von kleinem Durchmesser, so dass sie durch ein Punktionsloch in der Herzwand hindurchgeschoben werden kann.

Vorzugsweise ist der Innendurchmesser des abzweigenden Auslaßrohres etwa gleich groß wie derjenige der Kanüle. Auf diese Weise stellt sich in dem Auslaßrohr etwa die gleiche Strömungsgeschwindigkeit ein wie in der Kanüle. Die Abweichung sollte maximal etwa 10 % betragen. Hohe Strömungsgeschwindigkeiten in Rohr und Kanüle vermindern die Gefahr der Thrombenbildung selbst bei niedriger Antikoagulenz.

Vorzugsweise ist der Pumpenring zylindrisch ausgebildet, derart, dass das in ihm rotierende Pumpenrad eine Axialströmung erzeugt. Dies bedeutet, dass die Strömung im Bereich des Pumpenrades nicht oder nur sehr wenig in radialer Richtung abgelenkt wird, so dass die Außenkanten der Flügel des Pumpenrades eine zylindrische Umfangsfläche beschreiben. Dadurch werden unzulässig hohe Umfangsgeschwindigkeiten in kleinen Spalten am Pumpenrad vermieden, zumal der Durchmesser des Pumpenrings und der des Pumpenrades genau gefertigt werden können und ein axialer Vorsatz des Pumpenrads keine Veränderung des Spaltes bewirkt.

Gemäß einer zweckmäßigen Weiterbildung der Erfindung ist an dem Gehäuse ein Drucksensor zur Druckmessung an dem zu pumpenden Blut vorgesehen. Das Signal des Drucksensors wird zur Erkennung unterschiedlicher Betriebszustände und/oder zur Ermittlung des momentanen Volumenstromes ausgewertet. Es hat sich herausgestellt, dass ein einziger Drucksensor ausreicht, um den momentanen Volumenstrom zu ermitteln. Der jeweilige Druck steht in einer eindeutigen Beziehung zu dem Volumenstrom. Diese Beziehung kann durch Eichung ermittelt werden. Außerdem können durch Druckmessung außergewöhnliche Betriebszustände, wie beispielsweise eine Versperrung des auslaßseitigen Blutstromes oder des einlaßseitigen Blutstromes festgestellt.werden.

Im folgenden wird unter Bezugnahme auf die Zeichnungen ein Ausführungsbeispiel der Erfindung näher erläutert.

Es zeigen:
- Fig. 1: einen Längsschnitt durch die erfindungsgemäße parakardiale Blutpumpe,
- Fig. 2: eine atriale Implantation der Blutpumpe zur Rechtsherzunterstützung,
- Fig. 3: eine apekale Implantation der Blutpumpe zur Linksherzunterstützung, und
- Fig. 4: eine atriale Implantation der Blutpumpe zur Linksherzunterstützung.

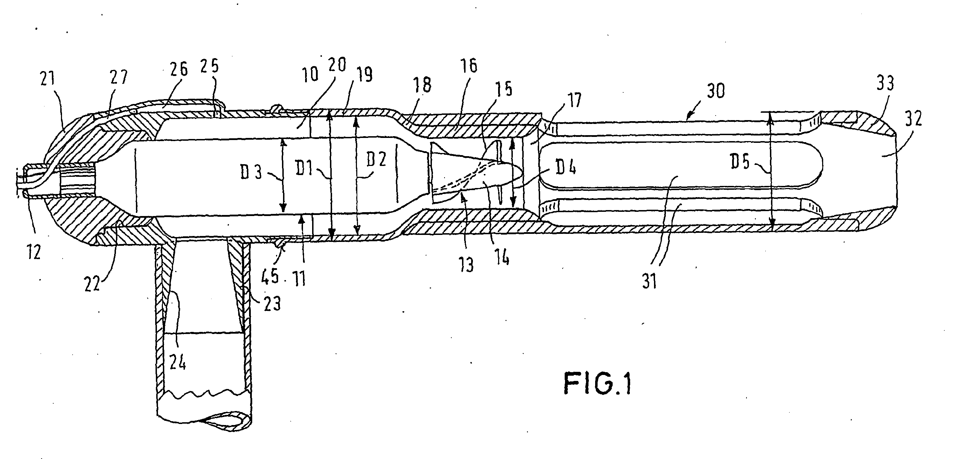

Die in Figur 1 dargestellte Blutpumpe hat ein längliches im wesentliches zylindrisches Gehäuse 10, in dem die Antriebseinheit 11 koaxial angeordnet ist. Die Antriebseinheit 11 enthält einen (nicht dargestellten) Elektromotor. Sie ist über elektrische Leitungen, die durch einen mit dem rückwärtigen Gehäuseende verbundenen Katheter 12 hindurchführen, mit einer elektrischen Steuer- und Versorgungseinheit verbunden. Auf einer Welle der Antriebseinheit 11 sitzt das Pumpenrad 13, das eine sich vom vorderen Ende aus konisch erweiternde Nabe 14 und davon abstehende Flügel 15 aufweist, welche schraubenförmig um die Nabe 14 verlaufen. Die Kanten der Flügel 15 bewegen sich auf einer Zylinderfläche, welche die Umhüllende des Pumpenrades 13 bildet.

Das Pumpenrad 13 rotiert in einem zylindrischen Pumpenring 16, dessen Durchmesser ungefähr gleich demjenigen der Umhüllenden des Pumpenrades ist. Hierbei beträgt der Laufspalt etwa 0,1 mm. Dies stellt einen guten Kompromiß zwischen Blutschädigung und hydraulischen Verlusten dar. Der Pumpenring 16 hat an seinem vorderen Ende einen axialen Einlaß 17, der hier aus einer umlaufenden Einführschräge besteht. An das rückwärtige Ende des Pumpenringes 16 schließt sich ein beschaufelungs- und rotorfreier Übergangsbereich 18 an, in welchem sich der Durchmesser von demjenigen des Pumpenringes auf denjenigen des die Antriebseinheit 11 umgebenden Gehäuseteils 19 kontinuierlich erweitert. Zwischen der Wand des Gehäuseteils 19 und der Außenfläche der Pumpeneinheit 11 ist ein ringförmiger Strömungskanal 20 gebildet.

Am rückwärtigen Ende des Gehäuses 10 befindet sich ein aus erhärtetem Klebstoff bestehender Haltekörper 21, der eine Ausnehmung 22 des Gehäuses ausfüllt und einen nach hinten vorstehenden gerundeten Ansatz bildet und der das rückwärtige Ende der Antriebseinheit 11 hält. Die Antriebseinheit 11 steht somit nach Art eines frei kragenden Auslegers von dem Haltekörper 21 ab, ohne dass eine seitliche Abstützung vorhanden wäre.

In der Nähe des rückwärtigen Endes des Strömungskanals 20 geht ein Auslaßrohr 23 seitlich von dem Pumpengehäuse ab. Dieses Auslaßrohr 23 verläuft tangential zu dem Strömungskanal 20. Seine Innenfläche 24 erweitert sich in Strömungsrichtung nach Art eines Diffusors mit einem Konuswinkel von maximal 8° zur Vermeidung von Ablösungen. An das Auslaßrohr 23 kann ein Schlauch mit glattem und stroßfreiem Übergang angeschlossen werden.

Im Bereich des Strömungskanals 20 ist an der Gehäusewand eine Druckerfassungsöffnung 25 vorgesehen, die mit dem Strömungskanal 20 in Verbindung steht. Von der Druckerfassungsöffnung 25 führt ein Druckkanal 26 in das Lumen eines Schlauchs 27, welcher durch den Katheter 12 hindurch verläuft. Am proximalen Ende des Katheters kann ein Drucksensor angeschlossen werden, um den Druck an der Stelle der Druckerfassungsöffnung 25 zu erfassen. Alternativ kann an der Druckerfassungsöffnung 25 ein Drucksensor installiert werden.

Die Druckerfassungsöffnung kann alternativ mit axialer Ausrichtung an der hinteren Stirnwand in unmittelbarer Nähe des Haltekörpers 21 angebracht sein oder mit radialer Ausrichtung an dem Pumpenring 16 im Bereich des Pumpenrades 13.

Der im Strömungskanal 20 herrschende Druck gibt Aufschluß über den momentanen Volumenstrom und auch über besondere Betriebsverhältnisse, wie beispielsweise eine Okklusion am Pumpeneinlaß oder am Auslaß. Die Überwachung dieser Parameter erfolgt mit einer einzigen Absolutwertmessung des Druckes, also ohne Differenzdruckmessung.

Das Gehäuse 10 ist mit einer rohrförmigen Kanüle 30 verlängert, welche auf den Pumpenring 16 befestigt ist. Die Kanüle 30 hat umfangsmäßig verteilt angeordnete längslaufende Schlitze 31 und am vorderen Ende eine axiale Öffnung 32 in einer abgerundeten Kappe 33. Die Länge der Kanüle 30 ist maximal gleich der Länge des Gehäuses 10, wobei der Pumpenring 16 dem Gehäuse zuzurechnen ist.

Der Außendurchmesser D1 des Gehäuseteils 19 ist bei dem beschriebenen Ausführungsbeispiel 11 mm. Der Innendurchmesser D2 im Bereich des Strömungskanals 20 beträgt 10 mm. Die Antriebseinheit 11 hat einen Durchmesser D3 von < 7,0 mm. Der Innendurchmesser D4 des Pumpenringes 16 beträgt 6 mm. Der Außendurchmesser D5 der Kanüle 30 beträgt 10 mm und der Innendurchmesser der Kanüle beträgt 8 mm.

Aus den genannten Abmessungen ergeben sich Blut-Strömungsraten im Bereich des Pumpenringes 16 von 1,5 bis 3 m/s, im Bereich des Strömungskanals 20 von 1,0 bis 1,5 m/s und im Bereich des Auslaßrohres 23 von 0,5 m/s. Die Antriebseinheit 11 läuft mit einer hohen Drehzahl von 25000 bis 30000 U/min. Hierbei fördert das Pumpenrad 13 4 bis 6 1 Blut pro Minute bei physiologischen Druckverhältnissen.

Das gesamte Gehäuse 10, einschließlich des Pumpenringes 16 hat eine Länge von etwa 50 mm und der über den Pumpenring hinaus vorstehende Teil der Kanüle 30 hat eine Länge von etwa 35 mm.

Die parakardiale Blutpumpe wird durch eine Punktionsöffnung in der Herzwand in das Herz derart eingeführt, dass das Gehäuse 10 die Punktionsöffnung abdichtend verschließt, während die Kanüle 30 sich im Innern des Herzens befindet und das Auslaßrohr 23 außerhalb des Herzens. Dabei ist die Punktionsöffnung in der Herzwand ohne Entnahme von Herzwandgewebe entstanden. Dies hat zur Folge, dass nach dem späteren Herausziehen der Pumpe die Öffnung in der Herzwand besser verschlossen werden kann.

Zur besseren axialen Fixierung der Pumpe an einer Herzwand mit einer sogenannten Tabakbeutelnaht befindet sich auf dem Gehäuse 10 eine umlaufende Verdickung 45.

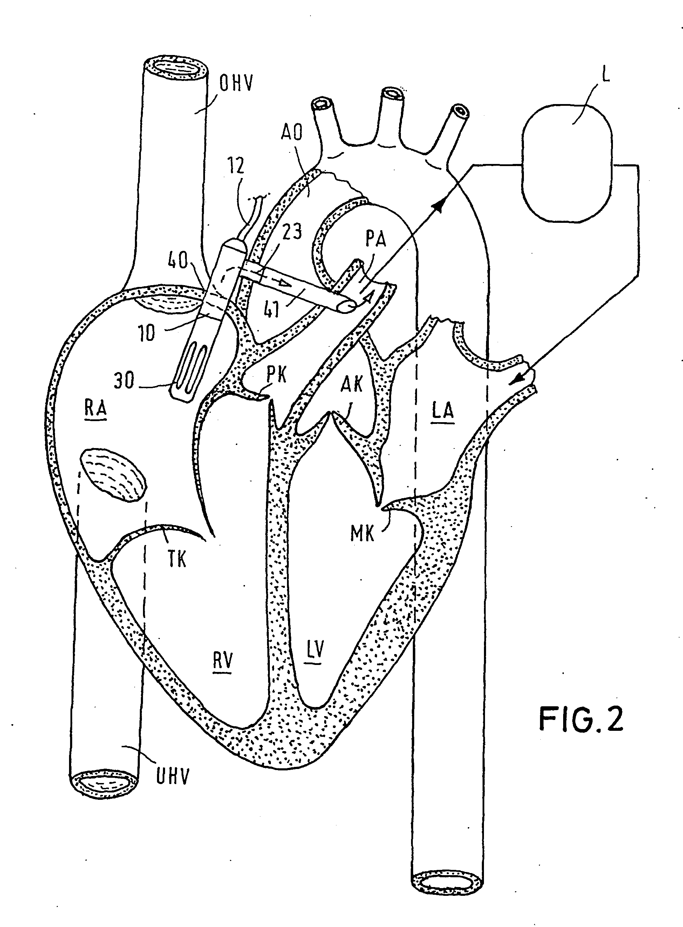

Figur 2 zeigt den atrialen Einsatz der parakardialen Blutpumpe bei der Rechtsherzunterstützung, wobei das Gehäuse 10 durch das rechte Vorhofohr 40 der Herzwand hindurch in das rechte Atrium RA ragt, während das Auslaßrohr 23 sich außerhalb des Herzens befindet und mit einem Schlauch 41 mit der Pulmonalarterie PA verbunden ist, welche das Blut vom rechten Ventrikel RV in die Lunge L leitet. Das von der Lunge L zurückkehrende Blut gelangt durch die Mitralklappe MK in den linken Ventrikel LV. Vom linken Ventrikel führt die Aortenklappe AK in die Aorta AO.

Zwischen dem rechten Atrium RA und dem rechten Ventrikel RV befindet sich die Tricuspidalklappe TK und zwischen dem rechten Atrium RA und der Pulmonalarterie PA befindet sich die Pulmonalklappe PK. In das rechte Atrium führen die obere Hohlvene OHV und die untere Hohlvene UHV.

Bei der beschriebenen Anordnung der Blutpumpe liegt ein wesentlicher Teil der Länge des Gehäuses 10 und die Kanüle 30 im Innern des Herzens, während ein relativ kurzer Teil des Gehäuses aus dem Herzen herausragt und das Auslaßrohr 23 zusammen mit dem angeschlossenen Schlauch sich eng an die Außenseite des Herzens anschmiegt. Daher nimmt die Blutpumpe keinen wesentlichen Platz ein.

Die Blutpumpe wird am offenen Herzen implantiert, um für die Zeit einer Operation oder eines anderen Eingriffs eine Herzunterstützung zu bewirken. Ein wesentlicher Vorteil besteht darin, dass im Brustbereich des Patienten keine schwergewichtigen und voluminösen Pumpen gelagert werden müssen.

Die Pumpe ist derart klein und leicht, dass selbst der fragile rechte oder linke Vorhof durch das Anlegen und Einbringen der Pumpe nicht wesentlich deformiert wird.

Bei dem in Figur 2 dargestellten Anwendungsbeispiel wird die Pulmonalklappe PK überbrückt. Das Herz schlägt im übrigen weiter, so dass die anderen Herzfunktionen in der gewohnten Weise ausgeführt werden.

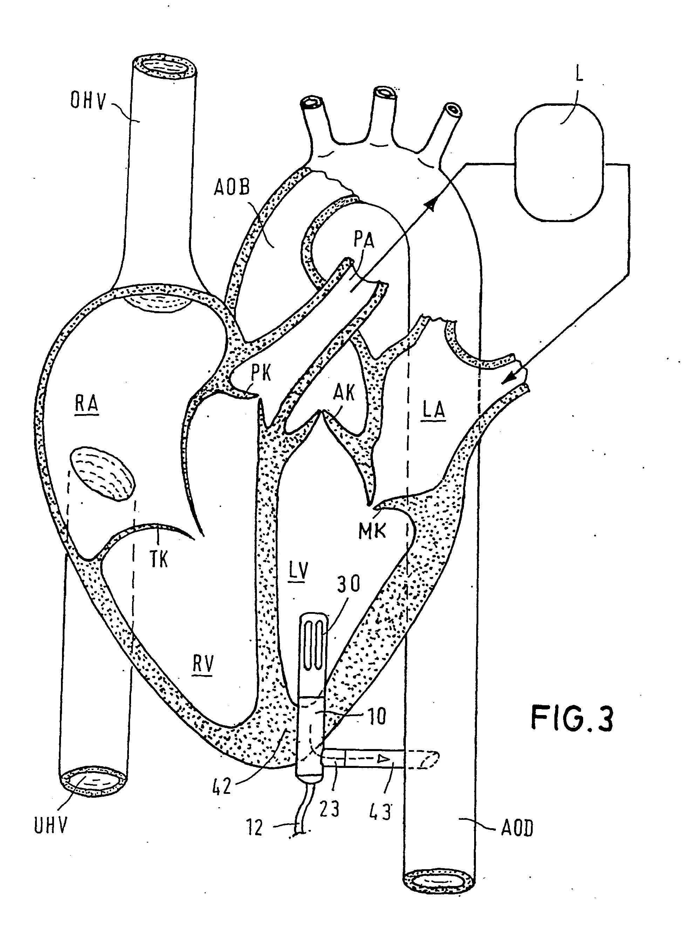

Figur 3 zeigt die Positionierung der Pumpe am Apex 42, also an der Spitze des linken Ventrikels LV. Auch hier ist das Gehäuse 10 von außen her durch ein Punktionsloch der Herzwand hindurchgeschoben, während das Auslaßrohr 23 mit einem angeschlossenen Schlauch 43 mit der Aorta Descendens AOD verbunden ist. Auch hier ist die räumlich gedrängte Anordnung der Pumpe auf engstem Raum unter geringstmöglicher Störung erkennbar. Die Pumpe fördert von dem linken Ventrikel LV in die Aorta Descendens und überbrückt dabei die Aortenklappe AK.

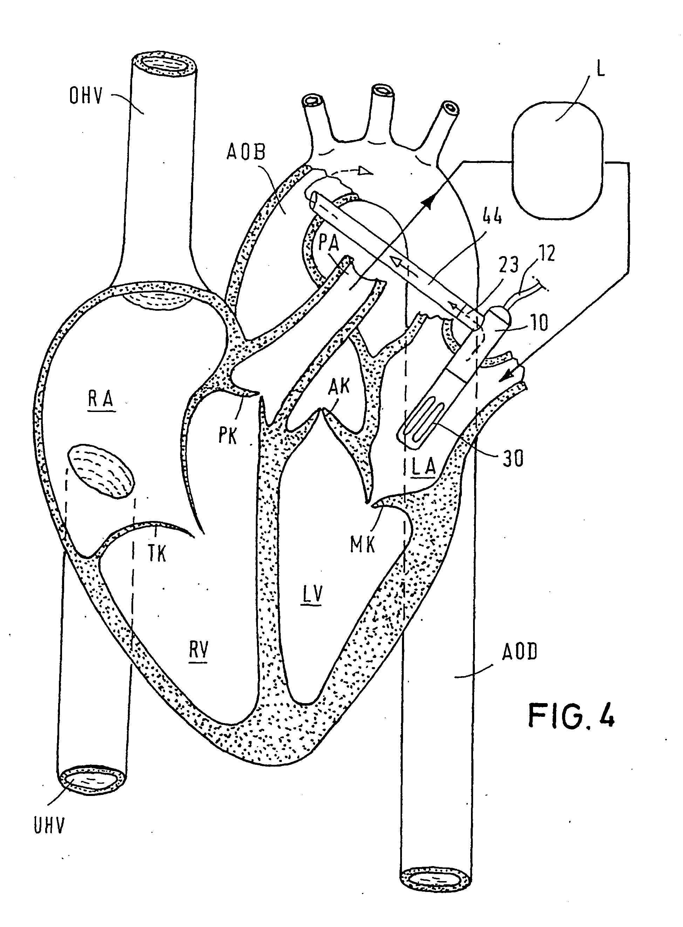

Bei dem Anwendungsbeispiel von Fig. 4 ragt das Gehäuse 10 von außen her in das linke Atrium LA hinein, während das außerhalb des Herzens liegende Auslaßrohr 23 über einen Schlauch 44 mit dem Aortenbogen AOB oder der Aorta Descendens verbunden ist.

Man erkennt, dass in allen Fällen die Positionierung der Pumpe raumsparend erfolgt, wobei die Störungen und Beeinträchtigungen des Zugangs zum Herzen möglichst gering gehalten werden.

## Patentansprüche

1. Parakardiale Blutpumpe mit einem rohrförmigen Gehäuse (10), das einen längslaufenden Strömungskanal (20) und eine koaxial darin angeordnete Antriebseinheit (11) für ein Pumpenrad (13) enthält und an einem Ende einen das Pumpenrad (13) umgebenden Pumpenring (16) mit axialem Einlass (17) und am anderen Ende ein seitlich abgehendes Auslassrohr (23) aufweist, und mit einer vor dem Einlass (17) des Pumpenringes (16) angeordneten Kanüle (30),
**dadurch gekennzeichnet,**
**dass** das Gehäuse (10) und die Kanüle (30) annähernd gleiche Außendurchmesser (D1) von maximal 13 mm haben, mit einer Abweichung zwischen den Außendurchmessern von maximal 15%, und dass das Gehäuse (10) eine umlaufende Verdickung (45) zur axialen Fixierung an der Herzwand aufweist.

2. Blutpumpe nach Anspruch 1, **dadurch gekennzeichnet, dass** der Innendurchmesser des Auslassrohres (23) etwa gleich groß ist wie derjenige der Kanüle (30).

3. Blutpumpe nach Anspruch 1 oder 2, **dadurch gekennzeichnet, dass** der Pumpenring (16) zylindrisch ist und in Strömungsrichtung hinter dem Pumpenring (16) ein Übergangsbereich (18) vorgesehen ist, dessen Durchmesser sich von demjenigen des Pumpenringes (16) auf denjenigen des Strömungskanals (20) erweitert.

4. Blutpumpe nach einem der Ansprüche 1 bis 3, **dadurch gekennzeichnet, dass** ein den Druck im Strömungskanal (20) erfassender Drucksensor vorgesehen ist und dass das Signal des Drucksensors zur Erkennung unterschiedlicher Betriebszustände und/oder zur Ermittlung des momentanen Volumenstromes ausgewertet wird.

5. Blutpumpe nach Anspruch 4, **dadurch gekennzeichnet, dass** der Drucksensor oder eine Druckerfassungsöffnung (25) an der Außenwand oder der axialen Endwand oder im Pumpenring (16) des Strömungskanals (20) angeordnet ist.

6. Blutpumpe nach einem der Ansprüche 1 bis 5, **dadurch gekennzeichnet, dass** die Kanüle (30) über den Pumpenring (16) hinaus um weniger vorsteht als die Gehäuselänge.

7. Blutpumpe nach einem der Ansprüche 1 bis 6, **dadurch gekennzeichnet, dass** der Strömungsquerschnitt im Bereich des Pumpenrades (13) so klein ist, dass bei Pumpenstillstand der Rückfluss durch die Pumpe kleiner ist als 1 l/min bei einem Gegendruck von 80 mm Hg.

8. Blutpumpe nach einem der Ansprüche 1 bis 7, **dadurch gekennzeichnet, dass** das Auslassrohr (23) einen sich in Strömungsrichtung erweiternden Querschnitt hat.

## Claims

1. A paracardial blood pump with a tubular housing (10) including a longitudinally extending flow channel (20) and a drive unit (11) for an impeller (13), said drive unit being arranged coaxially therein, and comprising, on the one end, a pump ring (16) with an axial inlet (17) surrounding the impeller (13) and, on the other end, a laterally branching outlet tube (23), and a cannula (30) arranged in front of the inlet (17) of the pump ring (16),
**characterized in**
**that** the housing (10) and the cannula (30) have approximately the same outer diameters (D1) of 13 mm at most, with a deviation between the outer diameters of 15% at most, and that the housing has a circumferential enlargement (45) for axial fixation to the cardiac wall.

2. The blood pump according to claim 1, **characterized in that** the inner diameter of the outlet tube (23) is about as large as that of the cannula (30).

3. The blood pump according to claim 1 or 2, **characterized in that** the pump ring (16) is cylindrical and that a transition region (18) is provided behind the pump ring (16) in flow direction, the diameter of said transition region enlarging from that of the pump ring (16) to that of the flow channel (20).

4. The blood pump according to one of claims 1 to 3, **characterized in that** a pressure sensor detecting the pressure in the flow channel (20) is provided, and that the signal of the pressure sensor is evaluated for the detection of different operating states and/or for the detection of the current volume flow.

5. The blood pump according to claim 4, **characterized in that** the pressure sensor or a pressure detection opening (25) is arranged on the outside wall or the axial end wall or in the pump ring (16) of the flow channel (20).

6. The blood pump according to one of claims 1 to 5, **characterized in that** the cannula (30) projects beyond the pump ring (16) by less than the housing's length.

7. The blood pump according to one of claims 1 to 6, **characterized in that** the flow cross section is so small in the region of the impeller (13) that the reflux through the pump is less than 1 l/min at a counter pressure of 80 mm Hg when the pump is at a standstill.

8. The blood pump according to one of claims 1 to 7, **characterized in that** the outlet tube (23) has a cross section enlarging in flow direction.

## Revendications

1. Pompe à sang paracardiaque, comportant un boîtier (10) tubulaire, qui comprend un canal d'écoulement (20) se développant en direction longitudinale et une unité d'entraînement (11) disposée coaxialement à l'intérieur, pour une roue de pompe (13), et présente, à une extrémité, une virole de pompe (16) avec une entrée axiale (17), entourant la roue de pompe (13), et, à l'autre extrémité, un tube de sortie (23) partant sur le côté, ladite pompe comportant encore une canule (30) disposée en amont de l'entrée (17) de la virole de pompe (16),
**caractérisée**
**en ce que** le boîtier (10) et la canule (30) ont des diamètres extérieurs (D1) approximativement identiques, au maximum de 13 mm, avec un écart entre les diamètres extérieurs de 15 % au maximum, et en ce que le boîtier (10) présente un épaississement circonférentiel (45) pour la fixation, en direction axiale, à la paroi du coeur.

2. Pompe à sang selon la revendication 1, **caractérisée en ce que** le diamètre intérieur du tube de sortie (23) est à peu près aussi grand que celui de la canule (30).

3. Pompe à sang selon la revendication 1 ou 2, **caractérisée en ce que** la virole de pompe (16) est cylindrique et, en aval de la virole de pompe (16), considéré dans la direction d'écoulement, il est prévu une zone de transition (18) dont le diamètre s'élargit de celui de la virole de pompe (16) à celui du canal d'écoulement (20).

4. Pompe à sang selon l'une des revendications 1 à 3, **caractérisée en ce qu'**un capteur de pression détectant la pression dans le canal d'écoulement (20), est prévu, et **en ce que** le signal du capteur de pression est exploité pour la reconnaissance d'états de fonctionnement différents et/ou pour la détermination du débit volumique instantané.

5. Pompe à sang selon la revendication 4, **caractérisée en ce que** le capteur de pression ou un orifice de détection de pression (25) est disposé sur la paroi extérieure ou la paroi d'extrémité axiale ou dans la virole de pompe (16) du canal d'écoulement (20).

6. Pompe à sang selon l'une des revendications 1 à 5, **caractérisée en ce que** la canule (30) fait saillie au-delà de la virole de pompe (16) sur moins que la longueur du boîtier.

7. Pompe à sang selon l'une des revendications 1 à 6, **caractérisée en ce que** la section transversale d'écoulement, dans la région de la roue de pompe (13), est suffisamment petite pour que, lors de l'arrêt de la pompe, le reflux à travers la pompe soit inférieur à 1 l/min, pour une contre-pression de 80 mm de Hg.

8. Pompe à sang selon l'une des revendications 1 à 7, **caractérisée en ce que** le tube de sortie (23) a une section transversale s'élargissant dans la direction d'écoulement.
